Europäisches Patentamt

European Patent Office

Office européen des brevets

(19)

(11) Veröffentlichungsnummer: **0 198 930**
**B1**

(12) **EUROPÄISCHE PATENTSCHRIFT**

(45) Veröffentlichungstag der Patentschrift: **04.07.90**

(21) Anmeldenummer: **85104733.2**

(22) Anmeldetag: **19.04.85**

(51) Int. Cl.⁵: **A 61 K 6/00,** A 61 K 6/10, A 61 K 6/02

(54) **Dentale Applikationsflüssigkeit zur Kontrolle von Zahnkontakten und prothetischen Arbeiten.**

(43) Veröffentlichungstag der Anmeldung:
**29.10.86 Patentblatt 86/44**

(45) Bekanntmachung des Hinweises auf die Patenterteilung:
**04.07.90 Patentblatt 90/27**

(84) Benannte Vertragsstaaten:
**AT BE CH FR GB IT LI LU NL SE**

(56) Entgegenhaltungen:
DE-C- 800 920
US-A-1 889 622
US-A-2 296 877
US-A-3 558 540

(73) Patentinhaber: **Prodent Ges. für zahnmed. Bedarfsartikel mbH**
**Frölingstrasse 16-18**
**D-6380 Bad Homburg (DE)**

(72) Erfinder: **Hansen, Jens Michael**
**Siegfried-Leopold-Strasse 6**
**D-5300 Bonn 3 (Beuel) (DE)**

(74) Vertreter: **Dost, Wolfgang, Dr.rer.nat., Dipl.-Chem. et al**
**Patent- und Rechtsanwälte Bardehle-Pagenberg-Dost-Altenburg & Partner Postfach 86 06 20**
**D-8000 München 86 (DE)**

EP 0 198 930 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Beschreibung

Die Erfindung betrifft eine neue Applikationsflüssigkeit für Dentalzwecke, die von Zahnärzten, Zahntechnikern und anderen in der Zahnheilkunde tätigen Personen verwendet werden kann, um Okklusionen (Kontaktpunkte) festzustellen und zu überprüfen, Approximalkontakte zu gestalten und zu kontrollieren und prothetische Arbeiten, einschließlich Inlays, Kronen, Brücken und dergl., und insbesondere auch die Paßgenauigkeit sämtlicher festsitzender prothetischer Arbeiten herzustellen und zu kontrollieren.

Es ist bekannt, zur Überprüfung und Herstellung der Paßgenauigkeit von festsitzenden prothetischen Arbeiten eine in Tuben abpackbare Masse auf Siliconbasis zu verwenden, die auf einem neutralen Untergrund wie einer Glasplatte oder einem Skalenblock mit einem Katalysator vermischt wird und dann als zähflüssige, elastische Masse meistens mittels eines Spatels in die Krone eingebracht wird. Anschließend setzt man die Krone auf den Stumpf und drückt sie fest; nach einer Abbindezeit von ca. 7 min. ist die Applikationsmasse hart geworden. Diese auf Siliconbasis zur Verfügung stehende Masse erfordert bei ihrer Handhabung einen erheblichen Zeit- und Arbeitsaufwand; außerdem läßt sie sich nur sehr schlecht dosieren.

Zur Überprüfung und Gestaltung von Approximalkontakten und der Okklusion sind bereits Okklusionspapiere in Form von größeren Folienflächen oder in Form von Bändern verschiedener Färbung vorgeschlagen worden, wobei die Farben die Stärke (Dicke) der Folien kennzeichnen sollen. Dicke Folien werden zur Grobgestaltung der Okklusionspunkte und dünnere Folien zur Feingestaltung der Okklusionspunkte und Artikulationspunkte (der Kontaktpunkte bei Lateral- und Vorschubbewegung des Kiefers) benutzt. Durch Zusammenbeißen der Zähne und durch Artikulationsbewegungen können die Kontaktpunkte festgestellt und überprüft werden. Für die Herstellung und Überprüfung von prothetischen Arbeiten sind derartige Okklusionspapiere jedoch ungeeignet.

Ferner sind sogenannte Okklusionswachse bekannt, die in Form von Plättchen vorliegen und zur Überprüfung des Zusammenbisses (Abduktion), d.h. der Okklusion, vorgeschlagen werden. Diese Okklusionswachse sind weder für die Feststellung der Artikulationsbewegung noch für die Herstellung und Überprüfung prothetischer Arbeiten geeignet.

Darüber hinaus sind sogenannte Okklusionssprays bekannt, die aus einer Spraydose geliefert werden und pulverförmige Konsistenz aufweisen, wobei die Körnigkeit der Pulver zwischen 10 und 20 μm liegt. Obgleich derartige Okklusionssprays bestimmte Handhabungsvorteile bieten, haften ihnen eine Reihe von Nachteilen an. Mit derartigen Okklusionssprays ist es kaum möglich, eine gleichmäßige Verteilung zu erreichen, da dann der Auftrag zu dick würde. Außerdem fehlt diesen Pulvern ein hinreichendes Haftvermögen an Metall, was zur Folge hat, daß das Pulver zwar auf dem Zahnstumpf haftet, jedoch eine ungenügende Haftung an den prothetischen Metallteilen zeigt. Die Druckstellen sind aus diesem Grunde sehr ungenau bestimmbar, da das Pulver auch bei anderen Partien schon bei geringstem Kontakt seine Haftfähigkeit verliert und deshalb eigentlich nur bei extremen Frühkontakten verwendbar ist. Ein weiterer Nachteil besteht darin, daß das Pulver bei dem unvermeidlichen Kontakt mit dem Speichel Fäden zieht und klumpige Ansammlungen bildet und dann unbranchbar wird. Die Entfernung dieses Okklusionssprays mittels Wasser ist nicht besonders gut zu bewerkstelligen. Beim Auftragen stäubt das Pulver sehr stark, so daß Finger, Hände und auch die nähere Umgebung stark verschmutzt werden.

Aus der US—A—2 296 877 ist eine breiartige Abdruckmasse zum Ersatz der bekannten Gips- oder Wachs-Gebissabdruckmassen bekannt, die aus einem Gemisch von Acrylharzen und Ethanol besteht. Diese Masse wird in üblicher Weise im Mund des Patienten um Zähne und Zahnfleisch herumgelegt, bzw. geknetet, worauf die Masse nach einigen Minuten, vermutlich aufgrund einer unvollständigen Polymerisation des eingesetzten Harzes, eine elastische Festigkeit erreicht.

Weiterhin ist in der DE—A—2 744 754 ein Verfahren und Mittel zum Kenntlichmachen von Kontaktstellen an Zahnersatz oder dgl. beschreiben. Dieses Mittel besteht aus dem eingetrockneten Milchsaft der Palaquium-Bäume, Trichlormethan sowie Magnesiumsilikat oder -oxid. Ein Nachteil dieses Kontaktmittels besteht in der sehr beschränkten Lagerfähigkeit, so daß es unmittelbar vor dem Auftrag auf den Zahnersatz aus den einzelnen Bestandteilen gemischt werden muß.

Der Erfindung liegt die Aufgabe zugrunde, eine gebrauchsfertige und deshalb unmittelbar applizierbare dünnflüssige, schnell trocknende und sowohl an Metall als auch an Zahnmaterial gut haftende Applikationsmasse bereitzustellen, die kontaktempfindlich, gut dosierbar, mit dem Speichel verträglich und dennoch leicht entfernbar ist und deren Handhabung und Auftragung einen möglichst ökonomischen Materialverbrauch gewährleistet.

Eine derartige Masse sollte für alle oben genannten Zwecke der Kontrolle und Überprüfung von Zahnkontakten und prothetischen Arbeiten geeignet sein.

Die Erfindung betrifft nun eine dentale Applikationsflüssigkeit, die Verwendung dieser Applikationsflüssigkeit für die Kontrolle von Zahnkontakten und zahnprothetische Arbeiten, sowie ein Verfahren zur Kontrolle von Zahnkontakten und bei zahnprothetischen Arbeiten, wie in den Patentansprüchen beschreiben.

Die erfindungsgemäße Applikationsflüssigkeit ist eine unmittelbar auftragbare Flüssigkeit, die im Gegensatz zu den oben erwähnten Massen auf Siliconbasis keine vorherige Zubereitung mittels eines Katalysators erfordert. Das Produkt ist dünnflüssig und läßt sich mittels Pinzette und Wattepellet ohne Schwierigkeiten auftragen und verteilen. Die Applikationsflüssigkeit ist schnell trocknend, ihre Trockenzeit beträgt nur ca. 5 bis 10 sec. und kann durch Anwendung eines Handpusters noch verkürzt werden. Die erfindungsgemäße Applikationsflussigkeit haftet auf allen Materialien, die in der Zahnheilkunde in Betracht kommen, einschließlich den Zahnmaterialien sehr gut und ermöglicht hierdurch eine außerordentlich genaue Feststellung von Druck- und Kontaktstellen. Die Markierung von Kontakten und Frühkontakten ist sehr deutlich. Aufgrund ihrer dünnflüssigen Konsistenz und ihrer Zusammensetzung ist die erfindungsgemäße Applikationsflüssigkeit gut und ökonomisch dosierbar; gegenüber den oben erwähnten Okklusionssprays wird deshalb eine wesentlich größere Anzahl von Kronenbestreichungen erreicht (Verbesserung ca. 350%). Die Flüssigkeit ist weder toxisch noch pulpairritierend. Nach Gebrauch läßt sich die Applikationsflüssigkeit von den Zähnen, Kronen und dergleichen gut und schnell mit einer Lösungsmittelflüssigkeit entfernen. Fädenbildung und Verklumpung aufgrund von Kontakten mit dem Speichel treten bei ihrer Anwendung nicht auf.

Die zahlreichen Vorteile der erfindungsgemäßen Applikationsflüssigkeit sind in der folgenden Tabelle den oben erwähnten Okklusionssprays gegenübergestellt.

## Tabelle

| | Okklusionsspray | Applikationsflüssigkeit der Erfindung |
|---|---|---|
| Aggregat | Pulver | flüssig |
| Löslichkeit in | Wasser: mäßig | Alkohol: gut |
| Körnigkeit | 10 - 20 μm | unter 10 μm |
| Trockenzeit | keine | 5 - 10 sec. |
| Dosierung | schwierig | gut |
| Haftbarkeit auf Metallen | ungenügend | gut |
| Verteilung | ungenügend | gut |
| Eigenschaften bei Speichelkontakt | Fädenbildung und Verklumpung | keine negativen Eigenschaften |
| Kontakterkennung | ungenügend, nur extreme Frühkontakte | gut |
| Sauberkeit | unsauber, große Staubentwicklung | sauber verwendbar |
| Anzahl der Kronenbestreichungen pro VE (Verpackungseinheit) | ca. 100 | ca. 350 |

Die erfindungsgemäße Applikationsflüssigkeit enthält in dispergierter bzw. gelöster Form ein organisches polymeres Bindemittel, dessen Körnigkeit kleiner als 10 μm ist. Vorzugsweise ist dieses Bindemittel ein natürliches Harz, insbesondere ein Harz aus der Gruppe der Schellackharze, Kolophoniumharze und modifizierten Kolophoniumharze. Eine andere geeignete Gruppe von Bindemitteln stellen die Alkydharze, Polyvinylacetale, Polyvinylalkohole, Polyvinylacetate, Polyacrylate und Ketonharz dar.

Der Anteil an organischem polymeren Bindemittel beträgt 0,5 bis 10 Gew.-%, insbesondere 1 bis 5 Gew.-%, jeweils bezogen auf Gesamtmasse.

Bei der massegebenden Komponente handelt es sich um einen pulverförmigen Bestandteil, der eine Körnung von unter 10 μm, vorzugsweise 5 bis 7 μm, besitzt. Vorzugsweise ist die massegebende Komponente aus der Gruppe der inerten, pharmazeutisch verträglichen Pigmente ausgewählt. Titandioxid, z.B. in Form von Titanweiß, ist ganz besonders bevorzugt. Titanweiß ist nach DIN 55 912 (Nov. 1960) ein Pigment, dessen farbbestimmender Anteil Titandioxid ist und das neben dem $TiO_2$ aus anstrichtechnischen

3

Gründen je nach Verwendungszweck zusätzlich Sulfate und Carbonate des Bariums, Calciums und Magnesiums aufweist, und zusätzlich bis zu 10% Zinkoxid enthalten kann. Der Titandioxid-Anteil liegt jedoch auf jeden Fall über 20%. Als $TiO_2$-Materialien kommen sowohl Anatas- als auch Rutilpigmente in Betracht.

Der Anteil der massegebenden Komponente in der Applikationsflüssigkeit beträgt 35 bis 65 Gew.-%, insbesondere 40 bis 60 Gew.-%, jeweils bezogen auf Gesamtmenge.

Zur visuellen Feststellung der Kontaktstellen enthält die erfindungsgemäße Applikationsflüssigkeit, ähnlich wie die oben erwähnten Silikonmassen, ein Buntpigment, das aus der Gruppe der physiologisch unbedenklichen Farbpigmente ausgewählt ist und eine grüne, rote, bevorzugt jedoch blaue Färbung bei Kontakten hervorruft. Bevorzugt werden Pigmente aus der Heliogenreihe verwendet, bei denen es sich um Buntpigmente auf Phthalocyaninbasis handelt. Ein besonders geeignetes Pigment ist Heliogen-Blau. Die Konzentration des Buntpigmentes richtet sich nach den Anwendungszwecken, wobei die Dosierung in üblicher Weise erfolgen kann. Der Buntpigmentgehalt liegt im Bereich von 0,01 bis 10 Gew.-%, insbesondere 0,1 bis 2 Gew.-%, jeweils bezogen auf Gesamtmasse.

Neben den oben erwähnten wesentlichen Komponenten der Applikationsflüssigkeit können gegebenenfalls weitere übliche Bestandteile von dentalen Applikationsmassen enthalten sein, die die Applikationsflüssigkeit antiseptisch, pyrogenfrei und steril halten.

Bei dem alkoholischen Lösemittel der Applikationsflüssigkeit handelt es sich bevorzugt um einen Alkohol, insbesondere Ethanol, kosmetischer Qualität oder reines Ethanol. Darüber hinaus können mit Alkohol andere physiologisch unbedenkliche Flüssigkeiten verwendet werden, solange sie die Konsistenz der Applikationsflüssigkeit nicht beeinträchtigen.

Somit ist die Zusammensetzung der Applikationsflüssigkeit wie folgt:

| Gewichtsteile | Allgemein | Bevorzugt |
|---|---|---|
| Bindemittel | 0,5 - 10 | 1 - 5 |
| massegebende Komponente | 35 - 65 | 40 - 60 |
| Buntpigment | 0,01- 10 | 0,1 - 2 |
| Lösemittel | 30 - 70 | 40 - 60 |

Es folgen Beispiele für einzelne bevorzugte Zusammensetzungen der erfindungsgemäßen Applikationsflüssigkeit, und jeweils ein Beispiel für deren Anwendung bei der Überprüfung der Okklusion, der Artikulation und bei der Überprüfung und Herstellung der Paßgenauigkeit von prothetischen Arbeiten.

## Beispiel 1
Herstellung der Applikationsflüssigkeit

3,63 Gewichtsteile Schellack (Gombal® OD 2) werden unter Rühren in 20 Gewichtsteilen Ethanol gelöst. Zu dieser Lösung werden unter Rühren jeweils 0,06 Gewichtsteile Bentone® W und Bentone® 34 (als Dispergierhilfsmittel), 48 Gewichtsteile Titandioxid (RN® 56) und 0,25 Gewichtsteile Heliogen-Blau zugesetzt. Die erhaltene Paste wird 30 Min. in einem Hochgeschwindigkeits-Dissolver dispergiert. Nach Erreichen der gewünschten Korngröße (<10 µm) wird die Restmenge Ethanol (28 Gewichtsteile) zugegeben. Man erhält eine dünnflüssige Applikationsflüssigkeit.

## Beispiel 2
Gezieltes Beschleifen der Innenflächen und Approximalflächen von Inlays, Kronen und dergleichen

Die Applikationsflüssigkeit von Beispiel 1 wird mittels Pinzette und Wattepellet gezielt auf Approximal- und Kauflächen appliziert. Nach einer Trocknungszeit von etwa 5 bis 10 sec. wird der prothetische Ersatz entsprechend eingesetzt, angedrückt und wieder entfernt.

Frühkontakte und somit Passungenauigkeiten werden an den Stellen sichtbar, an denen die aufgetrocknete Applikationsflüssigkeit verdrängt wurde bzw. fehlt. An diesen genau gekennzeichneten Stellen (Frühkontakte) sind nun zur Herstellung einer optimalen Paßgenauigkeit Schleifarbeiten notwendig (Abtragung der Frühkontakte).

Verschmutzungen an Händen und Geräten lassen sich mit einem alkoholischen Lösemittel, z.B. Ethanol, leicht entfernen.

## Beispiel 3
Überprüfung bzw. Korrektur (Einschleifen) der okklusalen Verhältnisse

Die Applikationsflüssigkeit von Beispiel 1 wird auf die Kauflächen der natürlichen Zähne appliziert.

Nach der Trocknungszeit läßt man den Patienten zusammenbeißen bzw. Artikulationsbewegungen durchführen (Lateralbewegungen). Die Okklusionskontakte bzw. Artikulationskontakte sind an den Stellen sichtbar, an denen die aufgetrocknete Applikationsflüssigkeit verdrängt wurde. Anhand dieser Punkte lassen sich einzelne Zahnkronen sowie das gesamte Gebiß gleichmäßig einschleifen (Herstellung einer ausgeglichenen Okklusion und Artikulation).

Beispiel 4

Gezielte Überprüfung der Bißhöhe bei Amalgamfüllung

Nach Legung der Füllung wird diese mit der Applikationsflüssigkeit von Beispiel 1 bestrichen. Nach der Trocknungszeit läßt man den Patienten zusammenbeißen. Sollten danach einzelne größere Kontaktflächen auf der Füllung sichtbar sein, müssen diese mit entsprechenden Schnitzinstrumenten verkleinert werden.

Beispiel 5

Gezielte Kontrolle und Gestaltung der Approximalfächen von Kronen, Brücken und dergleichen

Die Applikationsflüssigkeit von Beispiel 1 wird auf die gesamte Approximalfläche aufgetragen. Nach dem Antrocknen wird der prothetische Ersatz entsprechend eingesetzt. Nach Entfernung der Prothese läßt sich anhand der Größe und Umrißform der verdrängten Fläche die Kontaktfläche bzw. der Kontaktpunkt überprüfen und ggfs. durch Schleifen umgestalten.

**Patentansprüche**

1. Dentale dünnflüssige Applikationsflüssigkeit für die Kontrolle von Zahnkontakten und zahnprothetische Arbeiten, dadurch gekennzeichnet, daß sie dispergiert gelöst folgende Bestandteile enthält:
   (a) 0,5 bis 10 Gewichtsteile eines organischen polymeren Bindemittels
   (b) 35 bis 65 Gewichtsteile einer massegebenden Komponente
   (c) 0,01 bis 10 Gewichtsteile eines Buntpigments
   (d) 30 bis 70 Gewichtsteile alkoholisches Lösemittel,
   wobei die Feststoffe eine mittlere Körnigkeit von nicht über 10 µm besitzen.

2. Applikationsflüssigkeit nach Anspruch 1, dadurch gekennzeichnet, daß das Bindemittel ein Naturharz oder ein modifiziertes Naturharz aus der Gruppe Schellack-, Kolophonium- und modifizierte Kolophoniumharze ist.

3. Applikationsflüssigkeit nach Anspruch 1, dadurch gekennzeichnet, daß das Bindemittel aus der Gruppe der Alkydharze, Polyvinylacetale, Polyvinylalkohole, Polyvinylacetate, Polyacrylate und Ketonharze gewählt ist.

4. Applikationsflüssigkeit nach einem der Ansprüche 1 bis 3, dadurch gekennzeichnet, daß die massegebende Komponente Titandioxid enthält.

5. Applikationsflüssigkeit nach einem der Ansprüche 1 bis 4, dadurch gekennzeichnet, daß das Buntpigment ein Phthalocyanyupigment, insbesondere un blaues Phthalocyaninpigment ist.

6. Applikationsflüssigkeit nach einem der Ansprüche 1 bis 5, gekennzeichnet durch einen Feststoffgehalt von 30 bis 70 Gewichtsprozent.

7. Applikationsflüssigkeit nach einem der Ansprüche 1 bis 6, gekennzeichnet durch folgende Zusammensetzung in Gewichtsprozent, jeweils bezogen auf Gesamtmenge:
   (a) 1 bis 5% polymeres Bindemittel
   (b) 40 bis 60% massegebende Komponente
   (c) 0,1 bis 2% Buntpigment
   (d) 40 bis 60% Lösemittel.

8. Applikationsflüssigkeit nach Anspruch 7, dadurch gekennzeichnet, daß die Komponenten (a), (b), (c) und (d) folgende Bedeutung haben:
   (a) Shellack
   (b) Titandioxid
   (c) Heliogen-Blau
   (d) Ethanol.

9. Verwendung einer Applikationsflüssigkeit nach einem der Ansprüche 1 bis 8 für die Kontrolle von Zahnkontakten und zahnprothetische Arbeiten.

10. Verfahren zur Kontrolle von Zahnkontakten und bei zahnprothetischen Arbeiten, dadurch gekennzeichnet, daß man auf dentale Approximal- und Kauflächen, insbesondere Inlays, Kronen, Brücken, natürliche Zähne und Gebisse, eine dentale Applikationsflüssigkeit nach einem der Ansprüche 1 bis 8 aufbringt, die Flüssigkeit trocknen läßt und nach Durchführung bzw. Imitation des Gegenbisses die Kontaktstellen visuell feststellt.

**Revendications**

1. Liquide d'application dentaire fluide pour le contrôle de contacts dentaires et des travaux de prothèse dentaire, caractérisé en ce qu'il contient en dispersion ou en solution les composants suivants:

(a) de 0,5 à 10 parties en poids d'un liant polymère organique,

(b) de 35 à 65 parties d'un composant donnant de la masse,

(c) de 0,01 à 10 parties en poids d'un pigment coloré,

(d) de 30 à 70 parties d'un solvant alcoolique, les solides ayant une taille de particules moyenne n'excédant pas 10 µm.

2. Liquide d'application selon la revendication 1, caractérisé en ce que le liant est une résine naturelle ou une résine naturelle modifiée, choisie dans le groupe des résines de gomme-laque, des résines de colophane et des résines de colophane modifiées.

3. Liquide d'application selon la revendication 1, caractérisé en ce que le liant est choisi dans le groupe des résines alkyde, poly(acétal de vinyle)s, poly(alcool vinylique)s, poly(acétate de vinyle)s, polyacrylates et résines cétoniques.

4. Liquide d'application selon l'une des revendications 1 à 3, caractérisé en ce que le composant donnant de la masse contient du bioxyde de titane.

5. Liquide d'application selon l'une des revendications 1 à 4, caractérisé en ce que le pigment coloré est un pigment phtalocyanine, en particulier un pigment phtalocyanine bleu.

6. Liquide d'application selon l'une des revendications 1 à 5, caractérisé par une teneur en matière sèche de 30 à 70% en poids.

7. Liquide d'application selon l'une des revendications 1 à 6, caractérisé par la composition suivante, en % en poids, dans chaque cas par rapport à la quantité totale:

(a) de 1 à 5% de liant polymère

(b) de 40 à 60% de composant donnant de la masse

(c) de 0,1 à 2% de pigment coloré

(d) de 40 à 60% de solvant.

8. Liquide d'application selon la revendication 7, caractérisé en ce que les composants (a), (b), (c) et (d) ont les significations suivantes:

(a) gomme laque

(b) bioxyde de titane

(c) bleu héliogène

(d) éthanol.

9. Utilisation d'un liquide d'application selon l'une des revendications 1 à 8, pour le contrôle de contacts dentaires et des travaux de prothèse dentaire.

10. Procédé pour le contrôle de contacts dentaires et dans des travaux de prothèse dentaire, caractérisé en ce que l'on applique sur les faces proximales et masticatoires en particulier d'inlays, de couronnes, de bridges, de dents naturelles et de dentiers, un liquide d'application dentaire selon l'une des revendications 1 à 8, on laisse sécher le liquide et on détermine visuellement les points de contact après réalisation ou imitation du serrage de dents.

**Claims**

1. A dental application liquid of low viscosity for checking tooth contacts and dental prosthesis work characterised in that it includes the following components in dispersed or dissolved form:

(a) from 0.5 to 10 parts by weight of an organic polymer binding agent,

(b) from 35 to 65 parts by weight of a mass-giving component,

(c) from 0.01 to 10 parts by weight of a colour pigment, and

(d) from 30 to 70 parts by weight of alcohol solvent wherein the solid materials have an average grain size of not over 10 µm.

2. Application liquid according to claim 1 characterised in that the binding agent is a natural resin or a modified natural resin from the group consisting of shellac, colophony and modified colophony resins.

3. Application liquid according to claim 1 characterised in that the binding agent is selected from the group consisting of alkyd resins, polyvinyl acetals, polyvinyl alcohols, polyvinyl acetates, polyacrylates and ketone resins.

4. Application liquid according to one of claims 1 to 3 characterised in that the mass-giving component includes titanium dioxide.

5. Application liquid according to one of claims 1 to 4 characterised in that the colour pigment is a phthalocyanine pigment, in particular a blue phthalocyanine pigment.

6. Application liquid according to one of claims 1 to 5 characterised by a solids content of from 30 to 70% by weight.

7. Application liquid according to one of claims 1 to 6 characterised by the following composition in percent by weight, in each case in relation to the total amount:

(a) from 1 to 5% of polymer binding agent,

(b) from 40 to 60% of mass-giving component,

(c) from 0.1 to 2% of colour pigment, and

(d) from 40 to 60% of solvent.

6

8. Application liquid according to claim 7 characterised in that the components (a), (b), (c) and (d) mean the following:

(a) Shellack
(b) Titanium dioxide
(c) Heliogen blue
(d) Ethanol.

9. Use of an application liquid according to one of claims 1 to 8 for checking tooth contacts and dental prosthesis work.

10. A method of checking tooth contacts and in dental prosthesis work characterised in that a dental application liquid according to one of claims 1 to 8 is applied to dental approximal and chewing surfaces, in particular inlays, crowns, bridges, natural teeth and dentures, the liquid is allowed to dry and after biting together has been effected or imitated the contact locations are visually ascertained.